# EUROPEAN PATENT SPECIFICATION

(11) **EP 3 086 701 B1**
(45) Date of publication and mention of the grant of the patent: **18.07.2018**
(21) Application number: 14830694.7
(22) Date of filing: 17.12.2014
(51) Int. Cl.: A61B 1/00, A61B 18/14

(54) **INTEGRATED OPTICAL SYSTEM FOR A MICROENDESCOPIC APPARATUS**
INTEGRIERTES OPTISCHES SYSTEM FÜR EINE MIKROENDOSKOPISCHE VORRICHTUNG
SYSTÈME OPTIQUE INTÉGRÉ DESTINÉ À UN APPAREIL MICRO-ENDOSCOPIQUE

(30) Priority: 23.12.2013 IT TO20131059
(43) Date of publication of application: 02.11.2016
(73) Proprietor: Fondazione Istituto Italiano Di Tecnologia, 16163 Genova (IT)
(72) Inventor: ANTONINI, Andrea, 16163 Genova (IT); LIBERALE, Carlo, 16163 Genova (IT); FELLIN, Tommaso, 16163 Genova (IT)
(74) Representative: Vitillo, Giuseppe
(86) International application number: PCT/IB2014/067011
(87) International publication number: WO 2015/097598

(56) References cited:
- EP-A1- 0 682 910
- WO-A1-01/19235
- WO-A1-2009/087527
- WO-A1-2013/130464
- US-A1- 2009 054 791

## Description

### Technical field

The present invention relates to an integrated optical system for a microendoscopic apparatus.

### Background art

Microendoscopes are optical instruments used in the biological-medical field for *in vivo* observation of tissues at cellular level. The optical and mechanical characteristics of these instruments depend on the typology of the biological tissue of interest.

In recent years optical systems have been developed which, when appropriately associated with microscope objectives, can provide images with ultrahigh-resolution (in some cases less than one micron) to a depth of several millimeters within cavities or soft tissues. The diameter of these optical systems is generally smaller than 3 mm, particularly in the range of 0.3 mm to 2 mm.

In particular, optical systems of this kind are used in the neuroscience field in association with microendoscopic apparatuses, e.g. for studying the central nervous system at depths in excess of one millimeter, i.e. beyond the limits of non-invasive or scarcely invasive techniques (such as 2-photon microscopy).

Such optical systems generally include cylindrical GRIN lenses (i.e. lenses having a gradual refraction index along the cylinder's radial direction), possibly combined with other optical elements, such as spherical lenses or other elements having the same or a similar diameter as GRIN lenses. The combination of several different optical elements is typically used for increasing the numerical aperture (NA), and hence either the resolution, the length of the device being equal, or, vice versa, the length of the optical device, the resolution being equal.

The optical systems with increased numerical aperture for microendoscopic apparatuses created so far generally consist of combinations of GRIN lenses and GRIN lenses with spherical lenses, as described, for example, in patent publications EP 1 283 413 A1 and US 7,511,891 B2. However, GRIN lenses with high numerical aperture introduce significant aberrations, mainly of the spherical type, unlike longer GRIN lenses, which have a low numerical aperture (e.g. approx. 0.25). In general, due to the optical aberrations introduced by GRIN lenses and by spherical lenses, the optical systems that offer the best resolution are the shortest ones.

On the one hand, the problems associated with optical aberration have been faced by suitably selecting the profile of the GRIN lenses used in the optical system. On the other hand, they have also been mitigated by means of optical adapters, even external to the body of the endoscope, which can provide compensation for such aberrations (e.g. as disclosed by patent documents US 7,511,891 B2 and US 2009/054791 A1).

The creation of such optical systems having a significant length and a high resolution with negligible aberrations, if possible at all, requires a control on the manufacturing process that inevitably leads to costs that are significantly higher than those required by shorter optical systems having the same optical characteristics.

German company Grintech GmbH is currently offering an optical system for microendoscopic apparatuses that has a significantly high resolution, is 4.0 mm long, and has a diameter of 1.4 mm. This optical system has a numerical aperture of 0.8, with a range view field of 30 microns in diffraction-limit resolution conditions. However, the typical cylindrical shape and the small dimensions of this optical system make it difficult to handle. For example, tools - like clamps -, which are commonly used for supporting said optical system, will tend to slide around its smooth and curved surface.

A further drawback of such traditional optical systems is caused by the necessity of implementing thereon a corrective lens.

For this implementation, it is necessary to insert the lens while manufacturing the cylindrical body or to create the lens directly at one end of the optical system, which - due to these particular conditions - is a rather complex, delicate and costly operation.

Another drawback associated with prior-art optical systems for microendoscopic apparatuses is due to the difficulty in keeping the optical system in the desired position along the optical path, i.e. aligned with the axis of the microscope objective, while carrying out the optical measurements. This is, in fact, a necessary condition for being able to properly use the whole imaging system of the microendoscopic apparatus.

In this field's technical literature, the above-mentioned drawback appears to have been faced by clamping the optical system through suitable clamps, e.g. the Micro-V-Clamp tool developed by company Thorlabs Inc., fitted with elastically preloaded jaws which can clasp the optical system and which are movably and adjustably mounted on a microscope for aligning said optical system with the optical axis of the microscope objective. In addition to clamping, this problem is also tackled by using specific *holders* for coupling the optical system, typically through axial holes into which the latter is inserted and coupled by mechanical interference. In both cases, however, the length that can be used for introducing the optical system into the tissue to be examined decreases by a quantity at least equal to the thickness corresponding to the length of said optical system that is in contact with the jaws of the clamp or with the surface of the *holder.* When using a relatively short optical system, e.g. approx. 4 mm, together with the Micro-V-Clamp tool developed by company Thorlabs Inc. (the jaws of which are approx. 3 mm thick), the remaining usable length of the optical system will be 1 mm; in this case, therefore, 75% of the length of the optical system will remain unused. Of course, the loss of usable length of the optical system could be limited by reducing the thickness of the contact section, i.e. by using thinner clamps or holders; in such a case, however, the adherence of the optical system will be impaired, leading to easier rotation/oscillation of the same.

Another drawback related to the positioning of the optical system concerns the calculation of the correct distance between the microscope objective and the input surface (generally situated in a proximal position) of the microendoscopic optical system. An improper position of the optical system, corresponding to an undesired offset (in some cases even just a few microns) from the nominal distance, would imply a displacement of the focus point of the lens, resulting in significant degradation in the overall tissue observation performance.

In brief, the above-described tools make the optical system difficult to position and cause a performance reduction, because they change the configuration of the whole system by adding the microendoscope support system. Moreover, the focusing of the first surface of the optical system, supported by traditional systems, at the wavelength required by the microscope necessitates a lighting for said surface that is not always available in *standard* laboratory microscopes. Without the possibility of displaying the first surface of the optical system at the required wavelength, it is necessary to calibrate the difference between the distance of the microscope focus at the visible light and the distance of the focus at the working wavelength.

Further patent documents are also known in this field which relate to optical systems for microendoscopic apparatuses.

United States publication US 8,099,156 B1 describes an optical system based on a microprobe that facilitates imaging, *testing* and/or analysis of cochlear tissue. One of the variants described in said publication envisages *in vivo* insertion of an optical system (500µm - 1mm diameter) with GRIN microlenses into the cochlear tissue through a capillary structure, and tissue imaging by fluorescence microendoscopy. The capillary structure includes a metal plate perpendicular to the capillary axis, acting as a support for the entire optical system (inclusive of the capillary structure) within the tissue to be analyzed. According to this technical solution, the metal plate only functions as a mere mechanical support, in that it facilitates the positioning of the capillary structure relative to the microscope and to the material to be observed; however, since it is outside the optical path of the endoscope, it cannot by any means contribute to improving the optical characteristics of the image being transferred. Moreover, since said plate surrounds the cylindrical body of the probe, it shortens the length of the latter that can be used for in-depth inspection.

Patent publication WO 2013/082156 describes an optical system that can provide high-resolution imaging of thick biological tissue by using the so-called *"inverse direction"* method, without using any dye. The microendoscopic apparatus whereon the optical system is used can be designed as a manually controlled device. More in detail, the optical system comprises a microendoscopic probe for providing scarcely invasive tissue imaging, particularly at sarcomeric level for *in vivo* applications. The microprobe is supported and held in a stable position by means of a support, and can be coupled to or decoupled from the rest of the optical system by means of a clamp-type mechanism. In this case as well, the support is made of opaque material, and cannot therefore contribute to providing optical alignment with the microscope and improving the overall optical resolution of the system. Furthermore, the endoscope's clamp-type support reduces the usable length of the microendoscopic optical system that can be inserted into the tissue.

Patent publication US 7,302,142 B describes an integrated optical system for microendoscopic apparatuses which has the features mentioned in the preamble of the independent claim of the present invention. In particular, patent publication US 7,302,142 B concerns an optical system comprising a substrate, an optical component (in particular, an optical fiber), and a thin glass layer in contact with said optical component and said substrate. The thin layer, the thickness of which ranges from 0.1 µm to 10 µm, creates an optical connection between the optical component and the substrate, and can be configured for maintaining a spatial resolution of an endoscopic image. The substrate may be made from different materials, including glass, metal, semiconductor, plastic, or a shape memory material. In one variant, the optical component consists of GRIN microlenses. This technical solution, therefore, uses a thin glass layer acting as an optical connection between the optical component and the substrate. In short, the innovation described in said publication relates to the way in which different optical components for *imaging* applications are joined together.

However, the technical solution disclosed in patent publication US 7,302,142 B suffers from a few drawbacks.

One drawback is due to the necessity of creating a "stratified" structure including the substrate, the thin layer and the optical component, merged together at the interfaces, which translates into high costs and long times required for manufacturing the system.

Another drawback is due to the fact that the substrate, which may or may not be flat in shape, essentially plays a role merely as a mechanical support, without considering its optical or dimensional characteristics and its influence in terms of contribution to the optical path.

A further drawback is due to the fact that such a technical solution does not allow for quick and simple calculation of the distance to be kept between the optical system and the microscope objective.

Another important drawback derives from the fact that the application of a glass layer is envisaged in order to connect the support to the probe by welding, during which process high temperatures are typically reached to melt the vitreous layer. This expedient requires the use of heat-resistant materials for making both the support and the probe; in particular, this limits the possible options to materials having deformation (softening or melting) temperatures higher than those used for welding the glass layer onto the support, while also generally requiring that the support be considerably thick.

Document WO-A-01/19235 discloses an integrated optical system for a microendoscopic apparatus according to the preamble of claim 1.

### Summary of the invention

It is one object of the present invention to provide an integrated optical system for an endoscopic apparatus which can overcome the drawbacks of the prior art.

According to the present invention, this and other objects are achieved through an integrated microendoscopic optical system having the technical features set out in the appended independent claim.

It is to be understood that the appended claims are an integral part of the technical teachings provided in the following detailed description of the invention. In particular, the appended dependent claims define some preferred embodiments of the present invention which include some optional technical features.

One advantage of the present invention is that it provides an integrated optical system for microendoscopic apparatuses which is simple and easy to manufacture.

A further advantage of the present invention is that it provides an integrated optical system for microendoscopic apparatuses which is easier to handle and to align with a microscope.

An additional advantage of the present invention is that it provides an integrated optical system for microendoscopic apparatuses which allows exploiting the whole usable length of the optical system itself for sample examination.

Another advantage of the present invention is that it provides an integrated optical system for microendoscopic apparatuses whereon further functional components can be directly added in a simple and economic manner.

### Brief description of the drawings

Further features and advantages of the present invention will become apparent from the following detailed description, which is supplied by way of non-limiting example with particular reference to the annexed drawings, wherein:
- Figure 1 is a partial exploded perspective view of a microendoscopic apparatus including an integrated optical system made in accordance with one exemplary embodiment of the present invention;
- Figure 2 is a longitudinal elevation view of the apparatus shown in Figure 1;
- Figures 3 and 4 are, respectively, a perspective view and an elevation view of the system that is visible in the apparatus shown in the preceding figures;
- Figure 4a is an enlarged and partial schematic view of the system shown in Figure 4;
- Figures 5 and 6 are partial perspective views of, respectively, an apparatus according to one exemplary embodiment of the present invention and a rodent having cerebral tissue to be inspected by means of said apparatus;
- Figures 7 and 8 are further perspective views of the apparatus shown in Figures 1 and 2, wherein longitudinal and transversal translation mechanisms are also visible;
- Figure 9 is a side elevation view of an integrated optical system made in accordance with a further embodiment of the present invention and including a corrective lens;
- Figure 10 is a schematic side elevation view of a system similar to the one shown in Figure 9, representing the directions of some rays that cross the optical assemblies of the system; and
- Figure 11 is a three-dimensional diagram that shows the intensity distribution of the *point spread function* in the focal plane of the embodiment shown in Figure 10; and
- Figure 12 is a Cartesian diagram representing the elevation profile of a variant embodiment of a corrective lens applicable as an alternative to those included in the embodiments shown in Figures 9 and 10;
- Figure 13 is a side elevation view concerning an integrated optical system made in accordance with another embodiment of the present invention.

### Detailed description of the invention

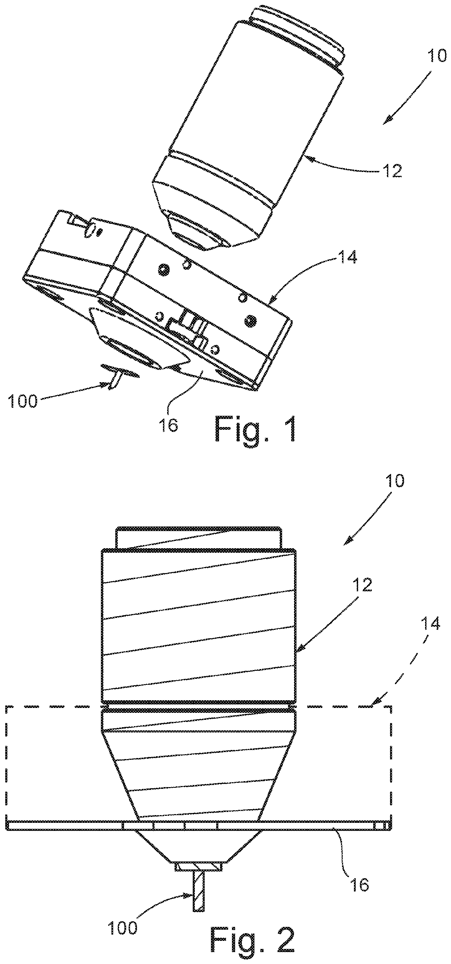

With reference to Figures 1, 2, 7 and 8, numeral 10 designates as a whole a microendoscopic apparatus comprising an integrated optical system 100 made in accordance with one exemplary embodiment of the present invention.

In particular, the apparatus comprises a microscope objective 12, a support 14, whereon microscope objective 12 is fixed, a frame 16, mounted at an adjustable distance from support 14 (and hence from microscope objective 12), and whereon system 100 is fixed. Some of the ways in which system 100 can be fixed to frame 14 will be described more in detail below.

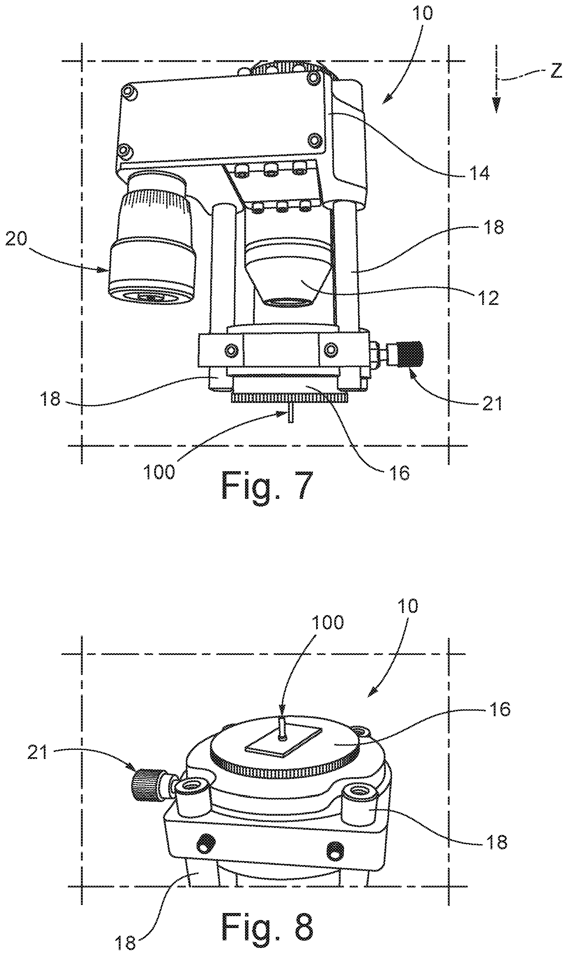

In the example shown in Figures 7 and 8, frame 16 is slidably mounted relative to support 14 on one or more longitudinal guides 18 (which in this case are mounted on support 14), particularly by means of a longitudinal adjustment mechanism 20, e.g. of the micrometric screw type. In this manner, by operating adjustment mechanism 20 one can adjust the distance along Z axis between system 100 and microscope objective 12 to select the focus thereof as desired.

System 100 comprises:
- a distal microendoscopic probe 102 having a prevalently longitudinal extension, capable of penetrating into a tissue to be observed, through which light radiations, directed towards the tissue and/or coming from said tissue, are able to pass; and
- a substantially plate-shaped proximal element 104 extending transversally to probe 102, wherein said element 104 can be coupled to a frame.

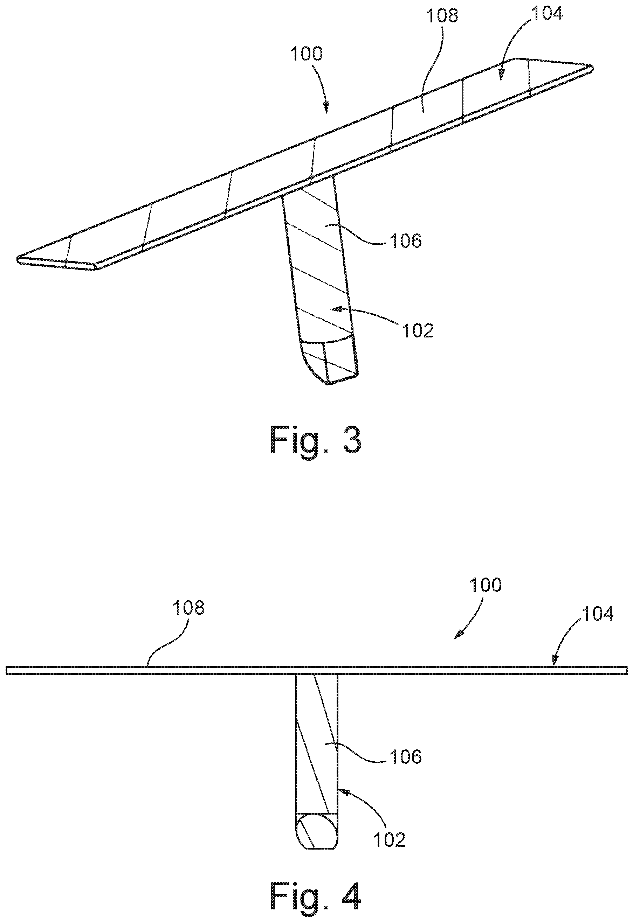

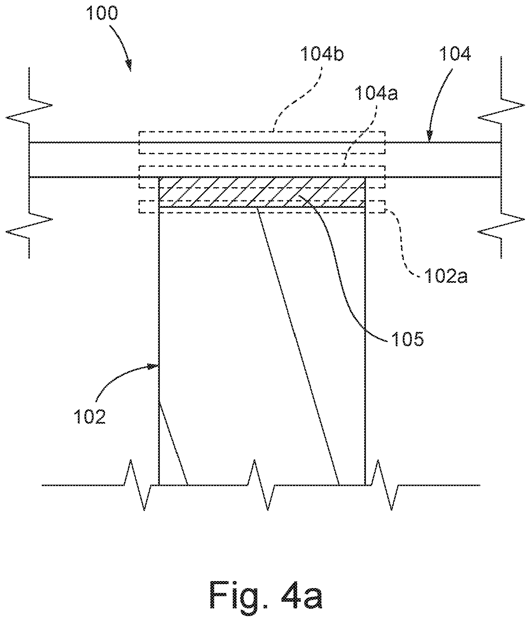

With particular reference to Figure 4a, element 104 is optically transparent at least in an input optical interface region thereof (i.e., by way of example, the part of planar surface of element 104 indicatively comprised in the dashed frame designated 104a) towards which the optically usable output area (i.e., by way of example, the part of planar surface of element 104 indicatively comprised in the dashed frame designated 102a) of probe 102 faces. Between the optically usable output area 102a of probe 102 and input optical interface region 104a of element 104 an optically transparent adhesive 105 is directly interposed, which constrains probe 102 securely to element 105.

As will be apparent to a man skilled in the art, Figure 4a is a qualitative view in which, in particular, the dimensions and proportions of the various components of system 100 should not be understood to be representative of the actual ones, since this drawing only aims at illustrating the principle of the invention.

Thanks to the above-mentioned features, system 100 can be easily handled, in particular by clasping element 104, thus avoiding the use of tools, such as clamps, for holding probe 102, which is typically uncomfortable and difficult to grip firmly for use. In fact, the absence of any such tools (clamps or *holders* with axial holes) allows exploiting substantially the whole longitudinal section through which probe 102 can penetrate into the tissue to be investigated.

In addition to affecting the handling of system 100, the above-mentioned technical features also favourably influence its general optical properties.

Therefore, according to the present invention, the performance of the device can be optimized by increasing the penetration length for certain optical resolution and section values of system 100. This advantage is particularly significant for systems 100 having a relatively short length, e.g. less than 10 mm.

In the illustrated embodiment, element 104 is optically transparent in the space comprised between its input optical interface region 104a and its output optical interface region (i.e., by way of example, the section of planar surface of element 104 indicatively comprised in the dashed frame designated 104b), i.e. the space that constitutes the optically active continuation of probe 102.

Element 104 can also be connected to external macroscopic support structures, typically borne by frame 16, on the surface opposite to that where probe 102 is located. Thus system 100 can be fully inserted into the cavity/tissue to be analyzed, avoiding - as aforementioned - any loss of observation depth due to clamps or other elements, such as *holders,* currently known in the art. In addition to this, direct manipulation of element 104 provides rapidity and simplicity when optically aligning system 10 by coupling the same element 104 to *standard* optomechanical components (e.g. those developed by company Thorlabs Inc.), which are macroscopic and flat (with flat surfaces having an area of the order of cm²).

In the embodiment shown in Figures 1 to 4, probe 102 comprises - and preferably consists of - an elongated body 106 through which light radiations can enter and exit.

Preferably, body 106 has a cylindrical shape, in particular a circular cross-section; body 106 may have - for example - a mean diameter smaller than 3 mm. Alternatively, body 106 may have different shapes, e.g. a prismatic shape, or a tapered profile, such as a conical shape, tapering off away from element 104. By way of example, in the conical shape case body 106 may have a mean diameter smaller than 3 mm.

In the illustrated embodiment, body 106 is associated and integral with element 104 comprising - and preferably consisting of - transparent material in at least the optically usable area. Moreover, the transversal extension of element 104 is greater than the mean transversal extension (or mean thickness) of probe 102, and hence of body 106.

In the illustrated embodiment, probe 102 (and particularly its body 106) is made of transparent material, or anyway a material allowing a light radiation to pass through it.

In the illustrated embodiment, body 106 may consist of glass or transparent polymeric material cylindrical in shape and having a variable refraction index along the radial direction of the cylinder body (GRIN), and having a length dependent on the refraction index distribution in the material, semispherical microlenses (the maximum diameter thereof being smaller than or equal to the diameter of the endoscopic body), or spherical or aspherical caps having a radius of curvature and a shape dependent on multiple factors. In particular, the most important ones of such factors are, for example, the light dispersion curve of the different materials employed and the particular optical performance required from the optical device in terms of maximum resolution and view field width.

Optionally, body 106 may include cylindrical bodies made of transparent glass or transparent polymeric materials, as well as empty spaces.

In the illustrated embodiment, within said probe 102 there are no monomodal or multimodal optical fibers, nor bundles of optical fibers.

In particular, probe 102 may consist of body 106 and - as will be described below with reference to further embodiments of the invention - a capillary element made of metal or glass or plastic that surrounds body 106.

Preferably, element 104 extends transversally to probe 102, being in particular positioned on one end thereof (corresponding, in this embodiment, to an optically usable area of said probe 102), e.g. peripherally widening around the proximal end of body 106.

As described above, element 104 is connected to probe 102 through an optically transparent adhesive layer 105, e.g. including a resin. The thickness of said layer can be calibrated at the assembly stage, in particular ranging from a value close to zero to a value of a few tens of microns. In the former case (value close to zero), probe 102 and element 104 are substantially brought in contact with each other, and adhesive 105, e.g. a resin, remains between probe 102 and element 104 only to fill the respective surface roughnesses; preferably, structural rigidity is ensured by the collar of adhesive 105 formed between the surface of element 104 and the end of probe 102. Should particular thermal and/or mechanical characteristics be required, so that it is appropriate to keep a thickness of some microns or tens of microns of material between probe 102 and element 104, this can be done by suitably dosing the quantity of optically transparent adhesive resin 105, and by positioning the two surfaces to be joined at the desired distance prior to activating the process of curing adhesive 105. Said curing may be induced, depending on the polymer in use, by ultraviolet light having a suitable wavelength or by specific thermal or chemical treatments.

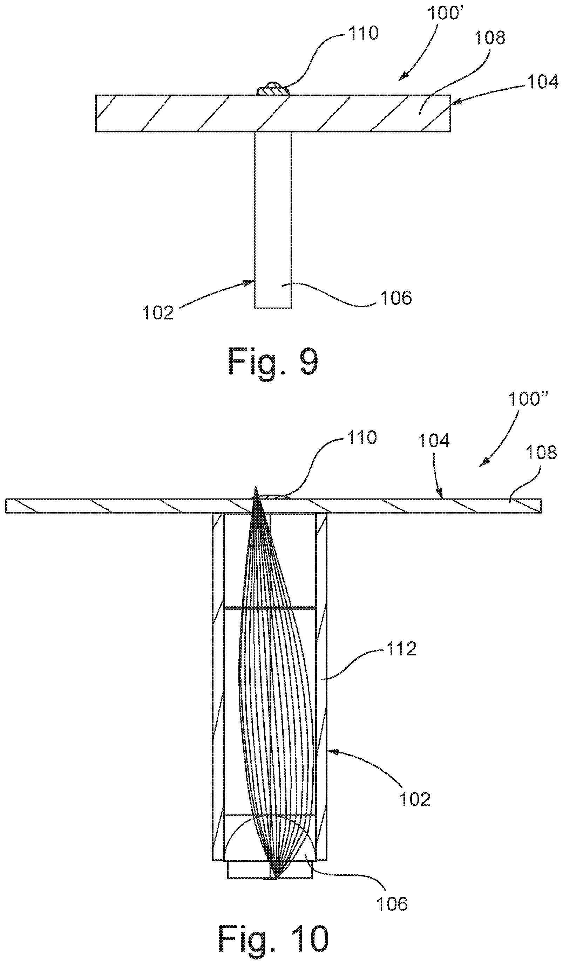

For reasons of mechanical strength (preferably not less than 80µm), the thickness of transparent element 104 may introduce a non-negligible optical stage when designing the optical elements of probe 102 and when defining the appropriate distance of element 104 from microscope objective 12. This element 104, preferably only consisting of the plate of transparent material or including an additional optical element on its surface (e.g. an aspherical corrective lens as shown in Figures 9,10), reduces the propagation of optical aberrations through system 100. In the illustrated embodiment, an optical aperture (i.e. an optically transparent region) of system 100 is on element 104; at said optical aperture, the transparent material preferably has a glossy surface. In particular, it is advantageous that said transparent material has a surface roughness at least smaller than one fourth of the wavelength at which system 100 will be operated.

Preferably, adhesive 105 comprises at least one resin selected from the group including acrylate, methacrylate, epoxy, acrylated urethane, methacrylated urethane or silicone-based resins.

In the embodiment shown in Figures 1 to 4, body 106 comprises - and preferably consist of - a lens with microfabricated aspherical curvature.

As aforementioned, element 104 is transparent and may preferably include at least one of a plastic material and a vitreous material. For example, said plastic material may include at least one of PMMA, polycarbonate, polystyrene, styrene, polymethylpentene (PMP), polyallyl-diglycol-carbonate (PADC). Element 104 may possibly comprise a combination of a plastic material and a vitreous material, which can anyway ensure transparency along the projection of probe 102 that intercepts said element 104.

Element 104 may also include materials of any other nature (whether metallic, non-transparent plastic, composite, with fluorescent *coatings*, opaque or reflecting) outside the space comprised between the output optical interface area 104b and the optically usable output area 102a of probe 102, i.e. the region participating in the transfer of the image from one end to the other of system 100.

In the embodiment shown in Figures 1 to 4, the thickness of element 104 is substantially constant. However, in further variants said thickness may be varied, in particular in order to improve the mechanical properties (e.g. higher mechanical strength) and/or the optical properties (e.g. better optical coupling with probe 102) of at least a portion of element 104. At any rate, the thickness of element 104 must be determined as a function of the optical properties required from system 100 and of the specific optical configuration of apparatus 10 (in particular, the optical characteristics of microscope objective 12) with which system 100 will have to be associated. As an alternative, the optical configuration of system 100 may be designed by assuming element 104 as an initial design constraint in terms of thickness and optical properties of the material, the characteristics of the other optical parts of probe 102 being defined accordingly.

With particular reference to Figure 4a, the thickness of element 104 - at least in the space comprised between its input optical interface region 104a and its output optical interface region 104b (i.e. the space that constitutes the optically active continuation of probe 102) - has a thickness preferably smaller than or equal to the transversal extension (in particular, the diameter) of probe 102, and in particular of body 106. For example, if probe 102 comprises a body 106 consisting of a GRIN glass cylinder having a diameter of 0.5mm, the thickness of the transparent layer located on top of probe 102, and belonging to component 104, will be smaller than or equal to 0.5mm.

In the embodiment shown in Figures 1 to 4, element 104 is mounted substantially perpendicular to the optical axis defined by probe 102, thus allowing a simple alignment of system 100 with microscope objective 12 (and hence with the optics of the microscope). By way of example, said alignment can be attained by simply ensuring parallelism between the flat surface of element 104 and the end of frame 16; this parallelism can be obtained, for example, by fixing the plane of element 104 to the end of frame 16 as previously mentioned (thereby attaining complanation) or, as an alternative to using a specific frame, by using other macroscopic optomechanical components having a flat external surface perpendicular to the optical axis of probe 102, thus reducing the possibility of mounting errors when in use.

In the illustrated embodiment, element 104 comprises a substantially flat plate 108, preferably having a constant thickness, made of transparent material, e.g. glass. Preferably, plate 108 is perpendicular to the optical axis defined by probe 102 (in particular, by body 106). Advantageously but not necessarily, plate 108 has a mean thickness of not less than approx. 0.08 mm, so as to ensure easy handling (e.g. as necessary for handling microscope cover slides classified as number 0) and to avoid direct contact of tools with probe 102, in particular with the aperture surface of optical body 106.

Preferably, plate 108 has a thin, substantially parallelepiped shape.

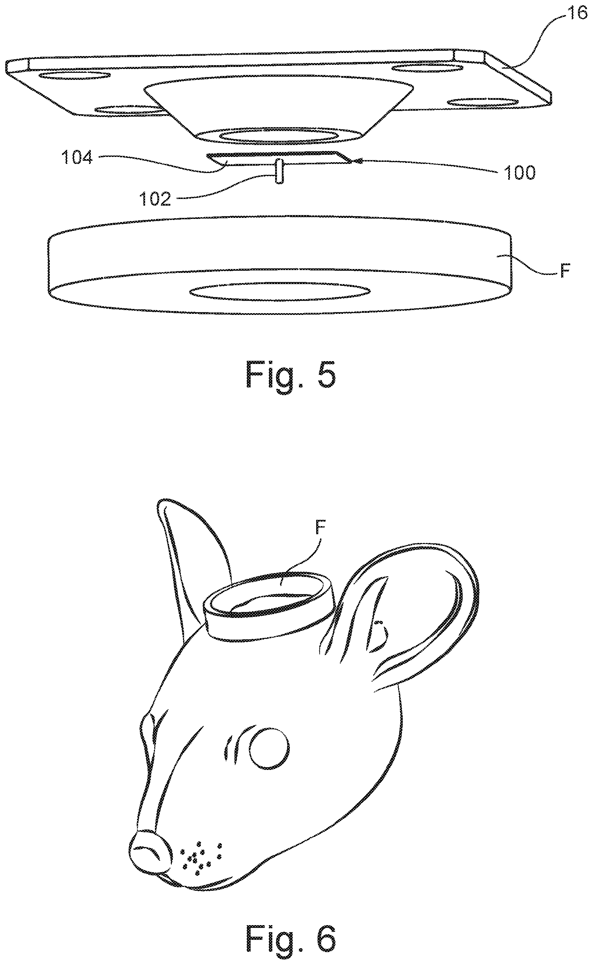

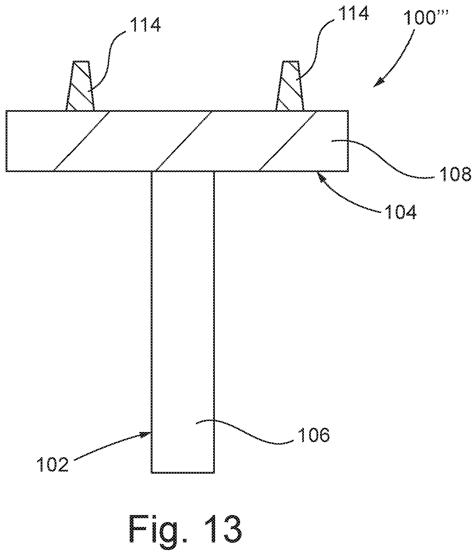

As represented by way of example in Figure 5, frame 16 may be connected to system 100 by glueing. In particular, said glueing may be carried out directly on mutually facing parallel surfaces of frame 16 and of element 104, as in the embodiment shown in Figure 5. In this way, system 100 will form a single optomechanical element, thus creating an integrated optical system supported by and promptly associable with support 14. Glueing may be obtained by using a removable or permanent adhesive, depending on the interchangeability requirements of the optical measurement system in use. According to an implementation variant, the two components can be fixed together by using magnetic foils connected to both surfaces to be joined. According to yet another variant embodiment, the connection can be obtained by providing plate 104 with mechanical reference components allowing the surface of plate 104 to be aligned with and secured to the bottom surface of frame 16, in which specific seats are provided for the insertion of such reference elements. By way of example, these elements may be cylindrical or conical guides with their axis perpendicular to the top surface of plate 104 (e.g. as shown in Figure 13), wherein the connection may be established by tightening a screw.

Positioning and centering system 100 with respect to frame 16 or another similar coupling system can be facilitated by specific reference guides designed on the frame or carved therein to a depth smaller than or equal to the thickness of element 104.

Merely by way of example, the following will describe a method of assembling probe 102 and element 104 together.

Once the components of system 100 have been defined in terms of thickness values, length values and materials of probe 102 and of element 104, such system 100 can be assembled together by using a micrometric handling system. In this case, probe 102, which is preferably cylindrical in shape, is positioned vertically in a plane that can be translated along 3 perpendicular spatial directions x,y and z, while element 104 is firmly positioned with its mating surface on top of and parallel to the flat surface of probe 102, at a distance that can be covered by the movement of the translation system.

The surface of probe 102 that has to be connected to the surface of element 104 is then wet with a transparent adhesive 105, and such surfaces are brought into contact with each other or to a desired distance by acting upon the vertical translation plane. In this condition, the optically transparent resin solidifies by undergoing a curing process induced by ultraviolet radiation or by thermal or chemical treatments.

The translatable plane that supports probe 102 is moved along x and y axes when element 104 includes an optical part that is sensitive to misalignment, e.g. a corrective lens created on the flat surface of transparent foil 108 (as shown in Figure 9), or fluorescent or non-fluorescent marking systems.

As can be seen in Figures 5 and 6, system 100 is versatile and can be adapted for use in many different research applications in accordance with specific user requirements, e.g. for dermatological investigations, subcutaneous inspections and neuroscience applications. In particular, said figures show a system 100 for studying rodent neurons. In this regard, Figure 6 shows a rodent on whose skull an aperture has been made, around which a frame F is located which acts as a perimetric containment zone for the physiological fluid. In this case, the use of a system 100 having a transversal dimension of element 104 (in particular, of plate 108) conveniently smaller than the transversal dimension of the axial through cavity defined by frame F allows effectively reaching the depth at which the rodent's cerebral tissue to be analyzed is located. This expedient allows exploiting the whole usable length of system 10, in particular without losing any part of the longitudinal extension of probe 102. In this context, in fact, the use of traditional tools (clamps or holders with axial holes) for constraining probe 102 would lead to a further limitation of the usable length of the probe, due to the height of frame F.

With reference to Figure 9, numeral 100' designates as a whole a system designed in accordance with a further exemplary embodiment of the present invention. Those parts or elements which are similar to or which perform the same function as those of the above-described embodiment have been assigned the same reference numerals. For simplicity, the description of such parts or elements will not be repeated below, and reference will be made to the above description of the previous embodiment.

In this embodiment, system 100' also allows fabricating a corrective lens 110 (e.g. having a height of less than a tenth of a millimeter) on element 104. In particular, corrective lens 110 is aligned with the optical axis of probe 102 and is fabricated on element 104. Corrective lens 110 is preferably positioned at a proximal end of system 100. In particular, corrective lens 110 performs the function of improving the optical coupling between system 100 and microscope objective 12.

In this embodiment, the position of corrective lens 110 allows it to be fabricated in a more simple manner: preferably, corrective lens 110 is obtained on element 104 (in particular, on plate 108) before joining element 104 to probe 102. The lens is thus fabricated on element 104, on the face thereof which is opposite to the one that is connected to probe 102, in particular opposed to the one from which body 106 protrudes. This mode of fabrication of corrective lens 110 makes the step of producing and aligning system 100 more efficient and economical than a direct implementation of the lens on probe 102 (in particular, on body 106).

With reference to Figure 10, numeral 100" designates as a whole a system designed in accordance with a further exemplary embodiment of the present invention. Those parts or elements which are similar to or which perform the same function as those of the above-described embodiments have been assigned the same alphanumeric references. For simplicity, the description of such parts or elements will not be repeated below, and reference will be made to the above description of the previous embodiments.

System 100" is applicable to a microendoscope with a punctiform light source *raytrace* offset relative to the optical axis, and includes a transparent element 104 provided with a corrective lens 110 on top of it (in particular, on the same side of plate 108 as in the preceding case).

With particular reference to Figure 10, probe 102 is preferably peripherally surrounded by a protection sleeve 112, in particular made of metallic material, which for example covers the outer surface of body 106. Of course, this measure can also be adopted in the other embodiments of the present invention described herein.

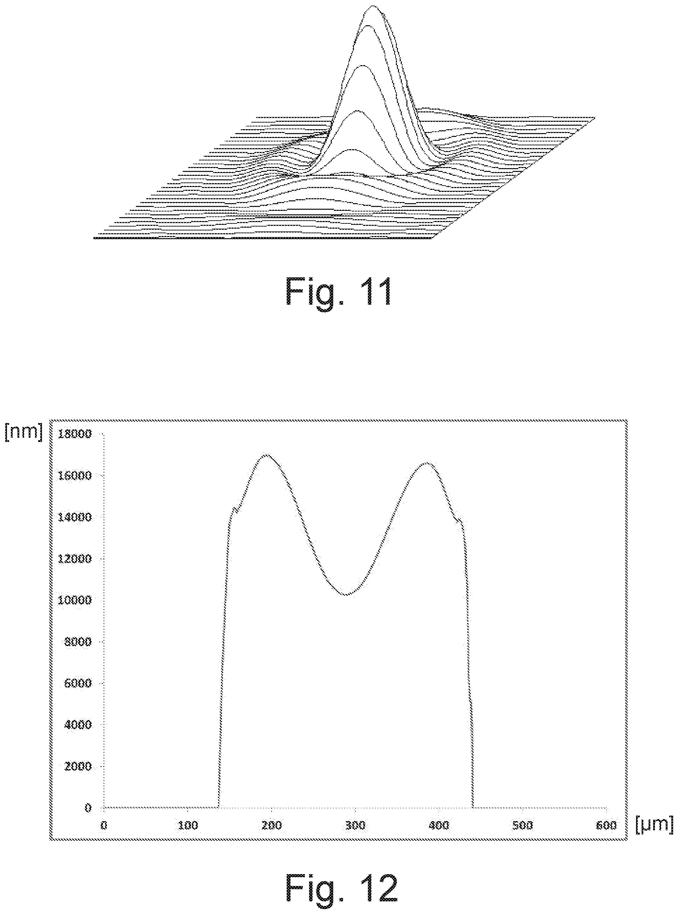

Figure 11 shows a three-dimensional diagram representing the intensity distribution of the *point spread function* in the embodiment shown in Figure 10. Said diagram relates, in particular, to the *point spread function* obtained for the configuration of system 100", wherein:
- probe 102 (in particular, its body 106) has a diameter of approx. 1 mm and a length of approx. 4 mm;
- transparent element 104 (in particular, its plate 108) is made of BK7 glass and is 0.15mm thick; and
- corrective lens 110 has an aspherical surface and a thickness of 40 microns.

Generally the resolution of such an optical system for microendoscopic apparatuses worsens significantly, especially along Z axis, as the number of so-called *pitches* (i.e. groups of *relays* that are present from one focal plane to another) increases, because each *point spread function* generated at the different focal planes along the optical path will be amplified, as shown in patent publication US 2009/0054791 A1. Therefore, the possibility of using an optical system made in accordance with the present invention, i.e. having a reduced longitudinal extension, particularly as far as the probe is concerned, allows keeping a *point spread function* having a limited width and, most importantly, a limited depth, without requiring an increased numerical aperture NA, thus maintaining a wide field of view.

Figure 12 represents a Cartesian diagram that shows the profile, viewed in elevation, of the surface of one implementation example of a corrective lens 110 applicable onto element 104, but different from those shown with reference to Figures 9 to 11. As can be appreciated, the scale of abscissas is in microns, whereas the scale of ordinates is in nanometers.

According to one advantageous implementation example, said corrective lens 110 is a lens microfabricated from polymeric resin having a refraction index of 1.56 at the 600nm wavelength, formed on glass that is 150 microns thick by using the 2-photon photolithographic technique. The particular shape of the surface is designed for correcting the aberrations of particular typologies of microendoscopes having a diameter of 0.5 mm. By way of example, the diameter of said lens is 300 microns, and its height is approx. 18 microns.

As aforementioned, such a corrective lens can be integrated on element 104 (e.g. on plate 108), in particular it can be carried by the face opposed to that from which probe 102 (e.g. body 106) protrudes, so as to improve the optical performance of system 100.

With reference to Figure 13, numeral 100'" designates as a whole a system designed in accordance with a further exemplary embodiment of the present invention. Those parts or elements which are similar to or which perform the same function as those of the above-described embodiments have been assigned the same alphanumeric references. For simplicity, the description of such parts or elements will not be repeated below, and reference will be made to the above description of the previous embodiments.

In this embodiment, system 100'" comprises one or more electrical and/or mechanical connectors 114 carried by element 104, preferably on the face opposed to that from which probe 102, particularly body 106, protrudes.

Preferably, said one or more connectors 114 are of the electrical type and allow the insertion of electrical connection components for connecting to additional devices and sensors, e.g. for a possible use of system 100'" for optoelectronic applications, which may be of particular interest in the neuroscience and optogenetics fields.

According to a further embodiment of the invention (not shown in the drawings), the transparent support of the optical system may include one or more portions provided with visible *marker* means, e.g. made of fluorescent material, outside the optically active area of the system for image formation. Through suitable visual references, this allows for simple focusing of the upper surface of optical system 100 (on the microscope objective side), also for one-photon or two-photon fluorescence measurements. Thanks to this expedient, therefore, bringing the distance between the optical system and the microscope objective to the desired value becomes a simple and fast operation. In the absence of transparent element 104, instead, it would not be possible to deposit the marker means (obtained, by way of example, from a fluorescent material) directly onto the input optical aperture of body 106 without introducing a fluorescent background that would reduce the signal-to-noise ratio of the optical system as a whole.

By way of example, the following will describe one possible way of using the marker means on the transparent element in order to facilitate the overall focusing process.

First of all, in order to bring the optical system into the correct measuring position it is necessary to change the distance between the objective and the microendoscopic system along the optical axis of the system, until a condition is achieved wherein the top surface of element 104 appears in focus. This process can be carried out by operating adjustment system 20 shown in Figure 7 (or another adjustment unit using a micrometric screw or a piezoelectric element). Once the distance has been found at which microscope objective 12 is in focus on the surface of foil 108, system 100 can be translated along the optical axis to the desired working distance (distance between the objective and system 100); this distance may vary for different configurations of probe 102 and, in general, is different from zero. By using a fluorescent marker made of a suitable material it is possible to find the distance at which the objective is in focus at exactly the desired wavelength. In the specific case of fluorescence induced by a two-photon microscope, the use of a fluorescent material allows said surface to be easily identified; in the absence of fluorescent material, instead, this surface will not be visible in the infrared range, and it will not be possible to easily determine the correct working distance for the optical system at the desired wavelength via direct optical measurement. Subsequently, while keeping constant this working distance between microscope objective 102 and element 104, it is necessary to translate system 100 in the plane perpendicular to the optical axis by means of adjustment mechanisms, e.g. like the micrometric-screw mechanism designated 21 in Figures 7 and 8, until the optical axis of the microscope objective coincides with the optical axis of system 100.

A fluorescent layer can be deposited onto component 104 by immersion into a fluorescent liquid material while masking the optical aperture of the system, or by depositing fluorescent particles around the optical aperture of the microendoscope. Particle deposition can be carried out, for example, by depositing an aqueous mixture, in which spherical fluorescent particles of polystyrene, melamine or another polymer having a diameter of less than one micron have been dispersed and diluted, onto the surface of component 104 and then letting the aqueous component of the mixture evaporate. In order to stabilize the fluorescent particles, it is preferable to coat the surface of element 104 with polylisine. During these processes, the optical aperture of microendoscopic system 100 needs to be covered to avoid obtaining a fluorescent background. Other marker material deposition processes may employ photolithographic techniques with resins charged with fluorescent molecules or may envisage glueing thin layers of fluorescent materials obtained by means of microfabrication techniques.

As will be apparent to a man skilled in the art, in accordance with the exemplary embodiments described and illustrated herein it is possible to obtain numerous and significant advantages from the use of the optical system of the invention, including, for example:
- possibility of using the whole length of the system (in particular, optimally exploiting the longitudinal extension of the probe) for insertion into tissues;
- reduced risk of soiling or spoiling the optically sensitive surfaces of the probe while handling the system (particularly when a reduced width is used, e.g. a diameter of less than 2-3 mm and typically within the range of approx. 0.3 to 2 mm), since only a direct action upon the lateral ends of element 104 is envisaged, without requiring any action upon the probe;
- rapidity and simplicity in focusing at the correct distance between the system and the microscope objective, in particular when visible marker means are used on the transparent support;
- reduction in the total microendoscopy measurement *setup* costs, due to the possibility of using either objectives without spherical aberration correction for use with cover slides on the sample to be inspected, or objectives with correction of aberrations induced by the thickness of the cover slide (by appropriately setting the thickness values and designing the optical parts); in biological laboratories the available objectives are often only of the type with optics that envisage the use of cover slides on the sample to be inspected, which, for microendoscopic measurements, should therefore be replaced with objectives not requiring said correction of aberrations introduced by the cover slide, to be specifically purchased for use with a microendoscope. For example, when using a configuration of system 100 with a plate 108 having a flat top surface, element 104 can be coupled to a cover slide made of borosilicate glass having the thickness specified by the microscope objective (generally in the thickness range of 0.1-0.2 mm) in a reversible manner (e.g. by using a layer of aqueous liquid) or in an irreversible manner (by using a layer of optical glue). The thickness of the glass to be coupled to component 104 will be greater than the one used in association with the objective in the absence of system 100 by a multiplicative factor generally comprised between 1 and 3; this factor will depend on the particular optical configuration of probe 102 in use. When considering the use of a system 100 comprising a corrective lens of the type shown in 110, there must be a cover made of transparent material for said lens, onto which a foil of transparent material may possibly be laid.
- possibility of using immersion objectives, since the surface on the microscope side (having dimensions of the order of cm²) is such that it allows depositing a drop of liquid (water or oil); and
- simpler, more efficient and less costly process of manufacturing and aligning the whole optical assembly, due to the modular fabrication of the system comprising probe 102 and element 104, which is even more important when using a corrective lens integrated into system 100 itself.

Of course, without prejudice to the principle of the invention, the forms of embodiment and the implementation details may be extensively varied from those described and illustrated herein by way of non-limiting example, without however departing from the scope of the invention as set out in the appended claims.

In particular, the technical characteristics that differentiate the various embodiments and variants thereof described and illustrated herein are freely interchangeable, whenever compatible. By way of non-limiting example, the corrective lenses envisaged in some embodiments of the present invention (e.g. those shown in Figures 9 and 10, wherein such lenses are designated 110) may be applicable to the embodiment shown in Figure 13 (in particular, the one using connector 114) and/or to the embodiment that includes the marker means (not shown). Furthermore, still by way on non-limiting example, an optical system may be conceived wherein a corrective lens, one or more connectors, and the marker means are simultaneously implemented in the support.

## Claims

1. Integrated optical system (100, 100', 100", 100'") for a microendoscopic apparatus (10), said system comprising:
- a distal microendoscopic probe (102) having a prevalently longitudinal extension, capable of penetrating into a tissue to be observed, through which light radiations, directed towards said tissue and/or coming from said tissue, are able to pass; and
- a substantially plate-shaped proximal element (104) extending transversally to said probe (102), said element (104) being capable of being coupled to a frame (16) of said apparatus (10);
wherein said element (104) is optically transparent in at least an input optical interface region (104a) towards which the optically usable area of said probe (102) faces;
said probe having an elongated body (106);
said system being **characterized in that** between the optically usable output area (102a) of said probe (102) and the input optical interface region (104a) of said element (104) an optically transparent adhesive (105) is directly interposed, which constrains said probe (102) securely to said element (104); and
that said probe (102) comprises a lens with microfabricated aspherical curvature;
said system further comprising a corrective lens (110) carried by said element (104), on the side opposed to that from which said probe (102) protrudes.

2. System according to claim 1, wherein said adhesive (105) comprises a resin, preferably of at least one type selected from the group consisting of acrylate, methacrylate, epoxy, acrylated urethane, methacrylated urethane or silicone-based resins.

3. System according to any one of the preceding claims, wherein said probe (102) comprises a protection sleeve (112) surrounding said body (106).

4. System according to any one of the preceding claims, wherein said element (104) is optically transparent in the space comprised between said input optical interface region (104a) and its transparent output optical interface region (104b).

5. System according to any one of the preceding claims, wherein said element (104) comprises a plate (108), the thickness of which is smaller than or equal to the transversal width of said probe (102), and which is preferably substantially perpendicular to the optical axis defined by said probe (102).

6. System according to any one of the preceding claims, further comprising at least one electrical and/or mechanical connector (114) carried by said element (104), preferably on the side opposed to that from which said probe (102) protrudes.

7. System according to any one of the preceding claims, wherein said element (104) includes at least one portion having visible marker means, preferably of fluorescent material, in a position spaced from said optical interface regions (104, 104b).

8. Microendoscopic apparatus (10) including a system (100, 100', 100'', 100'") according to any one of the preceding claims.

## Patentansprüche

1. Integriertes optisches System (100, 100', 100", 100'") für eine mikroendoskopische Vorrichtung (10), wobei das System umfasst:
- eine distale mikroendoskopische Sonde (102) mit einer geläufigen länglichen Verlängerung, die in ein zu beobachtendes Gewebe eindringen kann, durch das Lichtstrahlen, die auf das Gewebe gerichtet sind und / oder aus dem Gewebe kommen, passieren können; und
- ein im Wesentlichen plattenförmiges proximales Element (104), das sich transversal zu der Sonde (102) erstreckt, wobei das Element (104) mit einem Rahmen (16) der Vorrichtung (10) gekoppelt werden kann;
wobei das Element (104) in wenigstens einem optischen Eingangsschnittstellenbereich (104a), zu dem der optisch nutzbare Bereich der Sonde (102) weist, optisch transparent ist;
die Sonde (102) einen länglichen Körper (106) aufweist,
wobei das System **dadurch gekennzeichnet ist, dass** zwischen dem optisch nutzbaren Ausgangsbereich (102a) der Sonde (102) und dem optischen Eingangsschnittstellenbereich (104a) des Elements (104) ein optisch transparenter Klebstoff (105) direkt eingefügt ist, der die Sonde (102) sicher mit dem Element (104) verbindet; und dass die Sonde (102) eine Linse mit mikrohergestellter asphärischer Krümmung umfasst;
das System ferner eine Korrekturlinse (110) umfasst, die von dem Element (104) auf der Seite getragen wird, die derjenigen gegenüberliegt, von der die Sonde (102) vorsteht.

2. System nach Anspruch 1, wobei der Klebstoff (105) ein Harz umfasst, vorzugsweise von mindestens einer Art, ausgewählt aus der Gruppe bestehend aus Acrylaten, Methacrylaten, Epoxiden, acryliertem Urethan, methacryliertem Urethan, oder Silikon-basierten Harzen.

3. System nach einem der vorhergehenden Ansprüche, wobei die Sonde (102) eine Schutzhülse (112) aufweist, die den Körper (106) umgibt.

4. System nach einem der vorhergehenden Ansprüche, bei dem das Element (104) in dem zwischen dem optischen Eingangsschnittstellenbereich (104a) und seinem transparenten optischen Ausgangsschnittstellenbereich (104b) enthaltenen Raum optisch transparent ist.

5. System nach einem der vorhergehenden Ansprüche, wobei das Element (104) eine Platte (108) umfasst, deren Dicke kleiner oder gleich der Querbreite der Sonde (102) ist, und die vorzugsweise im Wesentlichen senkrecht zu der optischen Achse ist, die durch die Sonde (102) definiert ist.

6. System nach einem der vorhergehenden Ansprüche, ferner umfassend mindestens einen elektrischen und / oder mechanischen Verbinder (114), der von dem Element (104) getragen wird, vorzugsweise auf der Seite, die derjenigen gegenüberliegt, von der die Sonde (102) vorragt.

7. System nach einem der vorhergehenden Ansprüche, bei dem das Element (104) mindestens einen Abschnitt mit sichtbaren Markierungsmitteln, vorzugsweise aus fluoreszierendem Material, in einer von den optischen Schnittstellenbereichen (104, 104b) beabstandeten Position aufweist.

8. Mikroendoskopische Vorrichtung (10) mit einem System (100, 100', 100", 100'") nach einem der vorhergehenden Ansprüche.

## Revendications

1. Système optique intégré (100, 100', 100'', 100'") destiné à un appareil d'examen micro-endoscopique (10), ledit système comprenant :
- une sonde distale micro-endoscopique (102) présentant une extension principalement longitudinale, pouvant pénétrer dans un tissu devant être observé, à travers laquelle les rayonnements lumineux, dirigés vers ledit tissu et/ou provenant du tissu, sont capables de passer ; et
- un élément proximal (104) sensiblement en forme de plaque qui s'étend de manière transversale par rapport à ladite sonde (102), ledit élément (104) pouvant être accouplé à un cadre (16) dudit appareil (10) ;
dans lequel ledit élément (104) est optiquement transparent dans au moins une région d'interface optique d'entrée (104a) vers laquelle est tournée la zone optiquement utilisable (102a) de ladite sonde (102) ;
ladite sonde possédant un corps allongé (106) ;
ledit système étant **caractérisé en ce que**, entre la zone optiquement utilisable (102a) de ladite sonde (102) et la région d'interface optique d'entrée (104a) dudit élément (104), un adhésif optiquement transparent (105) est intercalé directement, qui fixe la sonde (102) solidement au dit élément (104) ; et
**en ce que** ladite sonde (102) comprend une lentille dotée d'une courbure asphérique micro-fabriquée ;
ledit système comprenant en outre une lentille correctrice (110) portée par ledit élément (104), sur le côté opposé à celui duquel fait saillie ladite sonde (102).

2. Système selon la revendication 1, dans lequel ledit adhésif (105) comprend une résine, de préférence d'au moins un type choisi dans le groupe consistant en les résines d'acrylate, de méthacrylate, d'époxy, d'uréthane acrylate, d'uréthane méthacrylate ou à base de silicone.

3. Système selon l'une quelconque des revendications précédentes, dans lequel ladite sonde (102) comprend un manchon de protection (112) qui entoure ledit corps (106).

4. Système selon l'une quelconque des revendications précédentes, dans lequel ledit élément (104) est optiquement transparent dans l'espace compris entre ladite région d'interface optique d'entrée (104a) et sa région transparente d'interface optique de sortie (104b).

5. Système selon l'une quelconque des revendications précédentes, dans lequel ledit élément (104) comprend une plaque (108), dont l'épaisseur est inférieure ou égale à la largeur transversale de ladite sonde (102), et qui est, de préférence, sensiblement perpendiculaire à l'axe optique défini par ladite sonde (102).

6. Système selon l'une quelconque des revendications précédentes, comprenant en outre au moins un connecteur électrique et/ou mécanique (114) porté par ledit élément (104), de préférence sur le côté opposé à celui duquel fait saillie ladite sonde (102).

7. Système selon l'une quelconque des revendications précédentes, dans lequel ledit élément (104) comporte au moins une partie porteuse de moyens marqueurs visibles, de préférence dans un matériau fluorescent, dans une position espacée desdites régions d'interface optique (104, 104b).

8. Appareil d'examen micro-endoscopique (10) comprenant un système (100, 100', 100", 100'") selon l'une quelconque des revendications précédentes.
